# EUROPEAN PATENT APPLICATION

(11) **EP 1 688 153 A2**
(43) Date of publication of application: **09.08.2006**
(21) Application number: 06250621.7
(22) Date of filing: 06.02.2006
(51) Int. Cl.: A61L 27/54

(54) **Infection resistant medical implants**

(30) Priority: 05.02.2005 GB 0502399
(71) Applicant: VASCUTEK LIMITED, Renfrewshire, Scotland PA4 9RR (GB)
(72) Inventor: Blackett, Barbara Mary, Paisley, PA2 7RL (GB); McNeil, George, Lanarkshire, ML5 5JH (GB)
(74) Representative: Ouzman, Beverley Nicola Claire

(57) **Abstract**

There is provided a medical device which is resistant to bacterial infection, especially *Staphylococcus* infection. The device is at least partially coated or impregnated with a composition comprising rifampicin and tobramycin. The medical device can be a vascular graft, vascular patch or a heart valve. The device can include 0.05 to 0.15mg/cm² of rifampicin and 0.02 to 0.05mg/cm² of tobramycin.

A method of making such a device is also described.

## Description

This invention relates to implantable medical devices, such as vascular grafts, patches and heart valves, which are rendered more resistant to bacterial infection by the incorporation of a combination of antibiotics.

Implantable medical devices made from synthetic materials are susceptible to infection. Whilst the infection rate is relatively low at less than 10% of patients, the consequences of infection at a site of implant can be catastrophic, with significant mortality and morbidity. In an effort to circumvent infection, it is routine practice to administer systemic, prophylactic antibiotics to these patients, but this does not always prevent infection. Synthetic biomaterials can be colonised by slime forming bacteria such as *Staphylococcus epidermidis,* which may not be accessible to the systemic drug as the bacteria are protected by the slime or biofilm.

In order to overcome this problem, many attempts have been made to incorporate antibiotic drugs into implanted devices.

Moore et al. (in Arch Surg (1981) 116:1403-1407) used collagen as a matrix to deliver amikacin from a vascular graft. Powell et al. describe combining rifampicin with the blood used to pre-clot a polyester vascular graft (Surgery (1983) 94(5):765-769). Other researchers bound oxacillin or penicillin to the surface of vascular grafts using surfactants. The surfactants used were benzalkonium chloride or tridodecylammonium chloride (TDMAC), which adsorb to the graft surface and then ionically bind the antibiotics (see Greco et al., Arch Surg (1985) 120:71-75 and Henry et al., J Thorac Cardiovasc Surg (1981) 82:272-277).

Rifampicin has also been loaded into a gelatin sealed vascular graft at the point of use, rather than incorporated during manufacture (see Goëau-Brissonnière et al., Annals of Vascular Surgery (1991) 5(5):408-412, and Journal of Vascular Surgery (1994) 19(4):739-744). The technique has been used clinically, and Strachan et al. as reported on its successful use in a series of patients (Eur J Vasc Surg (1991) 5:627-632).

Whilst these various approaches to loading or incorporating antibiotics have enjoyed some success, they also have limitations. Each approach relies on a single antibiotic drug. However all antibiotics have a limited spectrum of activity and so a single antibiotic may not be effective against the bacteria or strains of bacteria encountered in a particular patient. There is also the possibility of the emergence of resistance to a single antibiotic.

These limitations can be overcome by the use of a combination of antibiotics. A broader spectrum of activity will be achieved by using two antibiotics and the probability of resistance is also reduced.

US 5,217,493 and Raad et al. (Antimicrobial Agents and Chemotherapy (1995) 39(11):2397-2400) describe the use of two antibiotics, rifampicin and minocycline, bonded to the surface of medical devices. Whilst the use of a combination of rifampicin and minocycline has some advantages over a single antibiotic, this combination still has limitations. The purpose of the minocycline is to prevent resistance developing against the rifampicin, however, its activity is not particularly high. This antibiotic combination does exhibit a broader spectrum of activity compared to either antibiotic alone, but still does not have optimal activity, particularly against gram-negative organisms such as *Pseudomonas aeruginosa.* The bonding technique described in US 5,217,493 and by Raad et al. employs surfactants like TDMAC and thus introduces another material into the device, with possible adverse effects on biocompatibility.

We have now found that a combination of rifampicin and tobramycin can loaded onto a prosthetic graft during manufacture and provides an improved spectrum of activity.

The present invention therefore provides a medical device which is at least partially impregnated with an antibiotic composition or which has one or more of its surfaces at least partially coated with an antibiotic composition, said composition comprising a combination of rifampicin and tobramycin.

In one embodiment the medical device is for implantation with a patient. For example the medical device can be a prosthesis.

In one embodiment the medical device is a vascular graft, a vascular patch or a heart valve.

The medical device can be formed from any suitable material. Suitable materials include, but are not limited to, polyester, polytetrafluoroethylene (PTFE), expanded polytetrafluoroethylene (ePTFE), polyurethane, polypropylene or the like.

In one embodiment the medical device has been pre-treated with a sealant. Although the use of sealed graft is not essential, the use of a sealant avoids the need to pre-clot the graft. The pre-clotting process is inconvenient and might decrease the availability of the drug or interfere with its release.

Suitable sealants include, but are not limited to, gelatin, collagen, cross-linked dextran, polysaccharides (including cross-linked polysaccharides), albumin, hyaluronic acid, chitosan and synthetic hydrogels such as PVP, PEG and PEO.

In one embodiment the sealant is a gelatin sealant, which is optionally plasticised with a non-volatile non-toxic plasticiser. In one embodiment the plasticiser is glycerol, sorbitol or other suitable plasticiser. A suitable gelatin sealant is described in US 4,747,848 and in Drury et al., Ann Vasc Surg (1987) 1(5):542-547 (the contents of which are hereby incorporated by reference). This sealant has been in safe clinical use for many years and has been widely used for loading rifampicin at the point of use (see, for example, Strachan et al., (Eur J Vasc Surg (1991) 5:627-632).

This approach and particular combination of drugs overcomes many of the limitations of the prior art. No new materials are required to bind the drug. The spectrum of activity is very broad. Both antibiotics are highly active in their own right, and the combination is effective at suppressing resistance. The combination of the two drugs has also been unexpectedly discovered to have better activity against certain organisms than either alone. Thus the combination displays synergy.

Rifampicin can be introduced into the graft at a concentration of 1 to 20 g/L. Tobramycin can be introduced into the graft at a concentration of 0.5 to 12 g/L. The combination of rifampicin and tobramycin should be effective to inhibit the growth of bacteria responsible for infections, for example *Staphylococcus aureus.*

In one embodiment the antibiotic composition is present through substantially the whole thickness of the graft wall in at least one portion of the graft. Thus, at least one or more of the surfaces of the graft are impregnated with the antibiotic composition comprising rifampicin and tobramycin.

In one embodiment 0.05 to 0.15 mg (preferably 0.08 to 0.10 mg) of rifampicin is present per cm² of graft.

In one embodiment 0.01 to 0.05 mg (preferably 0.02 to 0.04 mg) of tobramycin is present per cm² of graft.

For implantable medical devices the most common infections are due to *Staphylococcus* bacteria. Indeed 70-80% of all such infections are due to *Staphylococcus aureus* and *Staphylococcus epidermidis.* In one embodiment the graft contains amounts of rifampicin and tobramycin effective against infections caused by *Staphylococcus* bacteria.

The preparation of a medical device according to the invention requires a relatively simple modification of the manufacturing process. The medical device is pre-treated with sealant and then can be simply soaked in a solution containing the two drugs. Rifampicin has limited solubility in water, and is preferably first dissolved in an alcohol. The sealant is generally plasticised, and it is important that the plasticiser (which may for example be glycerol) is not removed during the soaking step. Addition of the plasticiser to the soaking solution overcomes this problem.

In one embodiment the plasticiser is present as 60 to 90% (w/w) of the coating solution.

In a further aspect, the present invention provides a method of preparing an infection resistant medical device, said method comprising:
i) treating at least part of said medical device with a sealant to produce a treated medical device;
ii) exposing said treated medical device to a solution of rifampicin and tobramycin or to two solutions, one of which contains rifampicin and the other of which contains tobramycin.

The sealant used in step i) may be applied to the medical device in combination with a plasticiser as described above. Where the sealant is gelatin the plasticiser will generally be glycerol or sorbitol.

Optionally the solution(s) of step ii) may include a plasticiser, if a plasticiser is present in the sealant. Generally the same plasticiser would be used in both steps i) and ii).

In one embodiment the concentration of rifampicin in the solution of step ii) is 10 g/litre to 15 g/litre, preferably 11 g/litre to 14 g/litre.

In one embodiment the concentration of tobramycin in the solution of step ii) is 0.5 g/litre to 7 g/litre, preferably is from 1 g/litre to 6.5 g/litre.

The medical device must be sterilised before use and the sterilising process must be carefully selected to avoid inactivating the antibiotics. The use of low temperature (eg. 45°C or lower, for example 35-40°C) ethylene oxide gas as a sterilant has been shown to preserve the activity of the antibiotics.

The present invention also provides a method of inhibiting bacterial growth on a surface of a medical device, said method comprising:
a) applying a composition comprising a combination of rifampicin and tobramycin to at least part of a surface of said device, said composition being applied at a concentration sufficient to inhibit bacterial growth on the coated or impregnated surface.

The bacteria can be any *Staphylococcus* bacteria, for example can be *Staphylococcus aureus* or *Staphylococcus epidermidis.*

The present invention further provides the use of a composition comprising rifampicin and tobramycin to treat at least part of a medical device, in order to inhibit bacterial growth thereon. The medical device can be at least partially impregnated with the composition or can be least partially coated with the composition. Optionally the medical device has been pre-treated with a sealant and in one embodiment the sealant used is gelatine.
The present invention will now be further described with reference to the following, non-limiting, examples and figures in which:
Figure 1 shows the zone of inhibition in a culture of *Pseudomonas aeruginosa* on an agar plate containing a piece of graft coated with rifampicin and tobramycin.
Figure 2 shows the zone of inhibition in a culture of *Pseudomonas aeruginosa* on an agar plate containing a piece of graft coated with rifampicin and minocycline.
Figure 3 shows the zone of inhibition in a culture of *Escherichia coli* on an agar plate containing a piece of graft coated with rifampicin and tobramycin.
Figure 4 shows the zone of inhibition in a culture of *Escherichia coli* on an agar plate containing a piece of graft coated with rifampicin and minocycline.
Figure 5 shows the zone of inhibition in a culture of MRSA on an agar plate containing a piece of graft coated with rifampicin and tobramycin.
Figure 6 shows the zone of inhibition in a culture of MRSA on an agar plate containing a piece of graft coated with rifampicin and minocycline.
Figure 7 shows the zone of inhibition in a culture of *Staphylococcus aureus* on an agar plate containing a piece of graft coated with rifampicin and tobramycin.
Figure 8 shows the zone of inhibition in a culture of *Staphylococcus aureus* on an agar plate containing a piece of graft coated with rifampicin and minocycline.
Figure 9 shows the zone of inhibition is a culture of *Staphylococcus epidermidis* on an agar plate containing a piece of graft coated with rifampicin and tobramycin.
Figure 10 shows the zone of inhibition in a culture of *Staphylococcus epidermidis* on an agar plate containing a piece of graft coated with rifampicin and minocycline.
Figure 11 shows the zone of inhibition obtained following 2 days of blood recirculation through a graft containing rifampicin and tobramycin before exposure to bacteria:
   a) *Staphylococcus aureus*
   b) MRSA;
   c) *Staphylococcus epidermidis;*
   d) *Pseudomonos aeruginosa;* and
   e) *Escherichia coli.*
Figure 12 shows the differing concentration of alanine aminotransferase (ALT) following implantation of control grafts and grafts coated or impregnated with antibiotics respectively.
Figure 13 shows the differing concentration of aspartate aminotransferase (AST) following implantation of control grafts and grafts coated or impregnated with antibiotics respectively.
Figure 14 shows the differing concentration of alkaline phosphatase (AP) following implantation of control grafts and grafts coated or impregnated with antibiotics respectively.
Figure 15 shows the differing concentration of gamma glutamyl transferase (GGT) following implantation of control grafts and grafts coated or impregnated with antibiotics respectively.
Figure 16 shows the differing concentration of bilirubin (BIL) following implantation of control grafts and grafts coated or impregnated with antibiotics respectively.
Figure 17 shows the differing concentration of blood urea nitrogen (BUN) following implantation of control grafts and grafts coated or impregnated with antibiotics respectively.
Figure 18 shows the differing concentration of creatinine (CREAT) following implantation of controlled grafts and antibiotic grafts.

### Example 1

13g of rifampicin was added to 600 ml of isopropanol alcohol and 200 ml of glycerol in a flask. The resulting solution was then mixed on a rotary mixer for 20 minutes to ensure complete mixing.

In a separate flask, 1.7 g of tobramycin sulphate was added to 200 ml of deionised water. This solution was also mixed on a rotary mixer for 20 minutes.

The tobramycin sulphate solution was then added to the flask containing the rifampicin solution. This tobramycin/rifampicin mixture was then mixed for a further 20 minutes on the rotary mixer. After this time the tobramycin/ rifampicin solution was passed through a 0.45µm filter as a precaution to remove any particulate material. The final concentrations of antibiotics in this solution was 13 g/litre of rifampicin and 1.7 g/litre of tobramycin sulphate. These were the optimal concentrations, which would consistently give antibiotic concentrations of the substrate at or below the maximum daily dosage for each drug type. (Allowable limit for tobraymycin as specified in BNF 45 is 1 mg/kg up to 3 times daily. Accordingly the maximum amount of tobramycin applied to the graft is 210 mg (assuming an average human weight of 70 kg). Allowable limit for rifampicin as specified in BNF 45 is 600-1200 mg divided between 2 to 4 daily dosages. The minimum value is 600 mg in 4 doses, ie. a dose of 150 mg, which is the maximum amount applied to the graft.)

This solution was then used as a coating solution for either of the following:
a) Gelatin Sealed Polyester Vascular grafts and patches; and
b) Gelatin Coated expanded polytetrafluoroethylene (ePTFE) grafts.

Both grafts had been prior assessed to have an average uptake of 10ml solution.

The coating process for both these substrates is identical and described thus:-

The antibiotic solution was poured into a dip tank. The grafts to be coated were then placed into the dip tank with the antibiotic solution. The grafts were then agitated in the tank for 5 minutes, to ensure uniform coating of the graft with the antibiotic solution. After coating the grafts were then removed from the dip tank and dried by hanging vertically for 30 minutes. The grafts were then submerged in isopropanol for 20 seconds to remove excess glycerol from the graft. The grafts were then removed from the isopropanol and dried vertically for 24 hours. The grafts were then packaged and sterilised in ethylene oxide gas.

### Example 2

12 g of rifampicin was added to 600 ml of isopropanol alcohol and 200 ml of glycerol in a flask. The resulting solution was then mixed on a rotary mixer for 20 minutes to ensure complete mixing.

In a separate flask, 6.4 g of tobramycin sulphate was added to 200 ml of deionised water. This solution was also mixed on a rotary mixer for 20 minutes.

The tobramycin sulphate solution was then added to the flask containing the rifampicin solution. This tobramycin/rifampicin mixture was then mixed for a further 20 minutes on the rotary mixer. After this time the tobramycin/ rifampicin solution was passed through a 0.45µm filter as a precaution to remove any particulate material. The final concentrations of antibiotics in this solution was 12 g/litre of rifampicin and 6.4 g/litre of tobramycin sulphate. These were the maximal concentrations of antibiotics which would give the greatest benefit, but if released as a single bolus from the substrate could potentially exceed the maximum allowable daily dosage for tobramycin.

The substrates and method of coating are identical to the instructions in Example 1.

The grafts were then packaged and sterilised using ethylene oxide.

### Example 3

13 g of rifampicin was added to 800 ml of isopropanol alcohol in a flask. The resulting solution was then mixed on a rotary mixer for 20 minutes to ensure complete mixing.

In a separate flask, 1.7 g of tobramycin sulphate was added to 200 ml of deionised water. This solution was also mixed on a rotary mixer for 20 minutes.

The tobramycin sulphate solution was then added to the flask containing the rifampicin solution. This tobramycin/rifampicin mixture was then mixed for a further 20 minutes on the rotary mixer. After this time the tobramycin/ rifampicin solution was passed through a 0.45µm filter as a precaution to remove any particulate material. The final concentration of antibiotics in this solution was 13 g/litre of rifampicin and 1.7 g/litre of tobramycin sulphate. These were the optimal concentrations which would consistently give antibiotic concentrations on the substrate of, at or below the maximum daily dosage for each drug type. The final substrate will require plasticisation with glycerol.

This solution was then used as a coating solution for either of the following:
Gelatin Sealed Polyester Vascular grafts and patches; and
Gelatin Coated expanded polytetrafluoroethylene (ePTFE) grafts.

The coating process for both these substrates is identical and described thus:-
The antibiotic solution is poured into a dip tank. The grafts to be coated were then placed into the dip tank with the antibiotic solution. The grafts were then agitated in the tank for 5 minutes, to ensure uniform coating of the graft with the antibiotic solution. After coating the grafts were then removed from the dip tank and dried by hanging vertically for 30 minutes. The grafts were then submerged in a glycerol solution at 65°C for 5 minutes. After this time, the excess glycerol was removed from the graft by hanging vertically for 5 minutes. The grafts were then submerged in isopropanol for 5 minutes to remove the excess glycerol. The grafts were then dried vertically for 24 hours. The grafts were then packaged and sterilised in ethylene oxide gas.

### Comparative Example - Minocycline

A preparation of minocycline was prepared to give a concentration of 10 g/litre saline. This was the optional concentration, which would consistently give an antibiotic concentration of minocycline at or below the maximum daily dosage. (Allowable limit for minocycline as specified in BNF 45 is 100 mg twice daily. Accordingly the maximum amount of minocycline applied to the graft is 100 mg). This solution was used to coat grafts as described in Example 1.

Where a rifampicin/minocycline combination was to be applied to a graft, a solution was prepared with 12.5 g/litre minocycline and a solution of rifamplicin at 60 g/litre was prepared. 40 ml of the minocycline solution was combined with 10ml of the rifampicin solution to give the same concentrations of each antibiotic in the combined solution as for the individual solutions. The combined solution was applied to grafts as previously described.

### Example 4

Grafts (made according to Example 1) were evaluated by the zone of inhibition method. This involves placing pieces of graft onto agar plates, which have been inoculated with bacteria. An inoculum of 10⁶ bacteria was used for each agar plate. The treated graft samples were cut into equal lengths of 5 crimps. Each graft sample was laid on top of the incubated plate and placed in a 37°C incubator overnight. As the antibiotic diffuses from the graft it inhibits the growth of bacteria around the sample. A circular, clear area is formed, known as the zone of inhibition. Measuring the size of this zone permits quantification of the antibacterial effect of the sample. The zone around the graft sample was measured and photographed following overnight incubation.

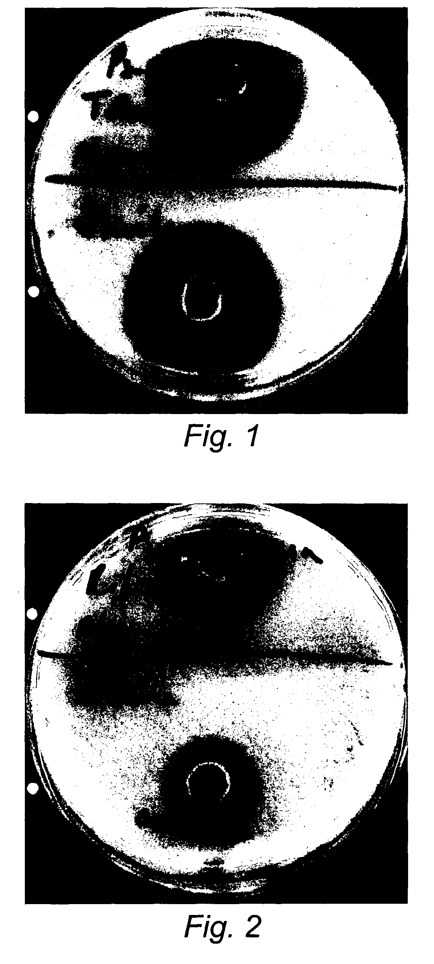

This testing has shown that the treated devices are effective against gram-negative and gram-positive organisms, that the combination is synergistic and more effective than rifampicin with minocycline.
The results are shown in Figures 1 to 4.
In Figure 1 a zone of inhibition can be seen around a rifampicin/tobramycin coated piece of graft. The zone size recorded was 28mm in diameter.

A similar zone of inhibition can be seen in Figure 2 which contains a rifampicin/minocycline coated piece of graft. However the zone size was only 10mm in diameter and is consequently much smaller than in Figure 1.

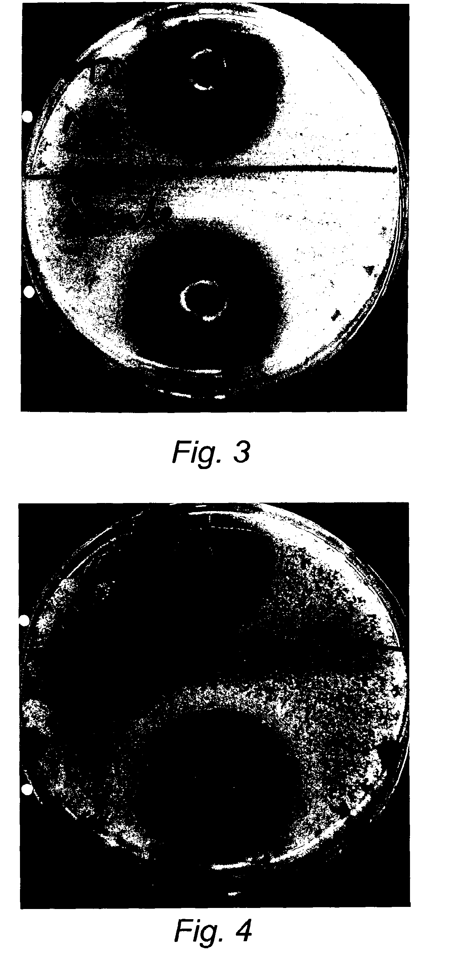

Thus greater activity was observed against *Pseudomonas aeruginosa* with the rifampicin/tobramycin combination than with the rifampicin/minocycline combination. Similarly, in Figure 3 a zone of inhibition can be seen around the rifampicin/tobramycin coated piece of graft. The zone size recorded was 22mm in diameter.

Whilst an equivalent zone of inhibition can be seen around the rifampicin/minocycline coated piece of graft in Figure 4, the zone size is smaller than in Figure 3 with a recorded zone size of 16mm in diameter.

Better activity was observed against *Escherichia coli* with rifampicin/ tobramycin combination than with rifampicin/minocycline combination.

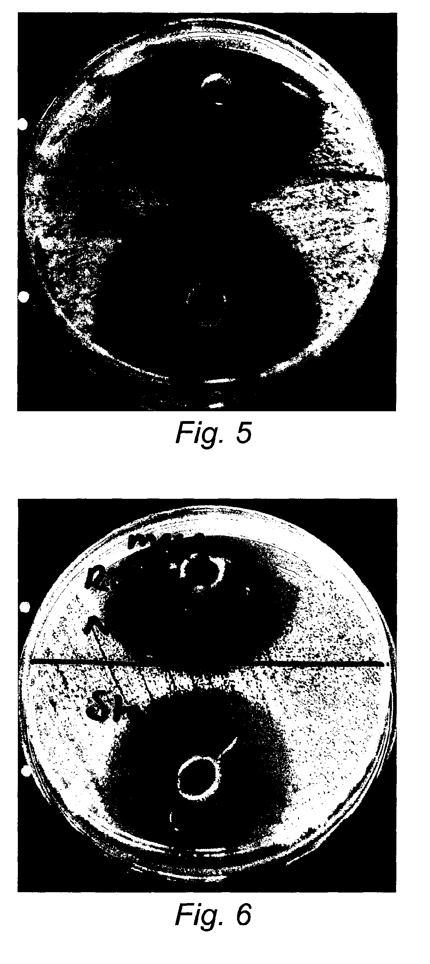

Figures 5 and 6 show a culture of MRSA on agar plates. A zone of inhibition can be seen around each coated piece of graft. Figure 5 shows a graft coated with rifampicin/tobramycin. The zone size recorded was 46mm in diameter. Figure 6 shows a graft coated with rifampicin/minocycline. The zone size recorded was 36mm in diameter.

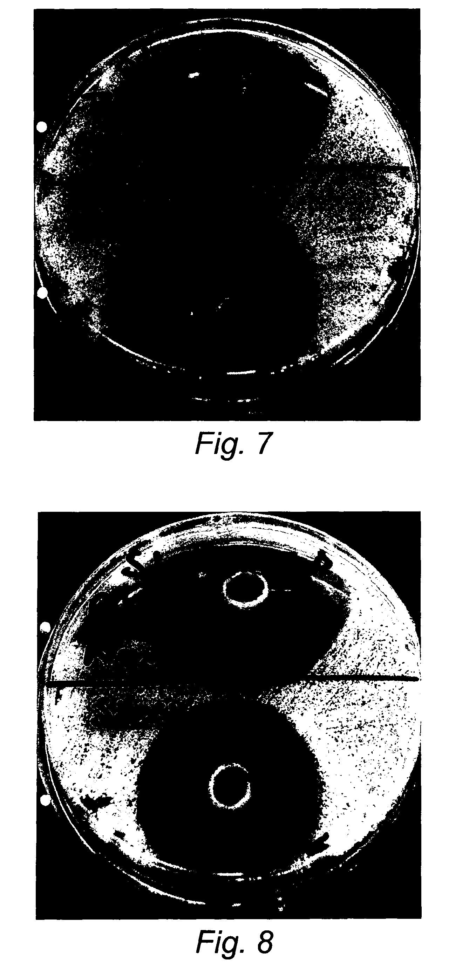

Figures 7 and 8 show a culture of *Staphylococcus aureus* on agar plates. A zone of inhibition can be seen around each coated piece of graft. Figure 7 shows a graft coated with rifampicin/tobramycin.
The zone size recorded was 36mm. Figure 8 shows a graft coated with rifampicin/minocycline. The zone size recorded was 28mm.

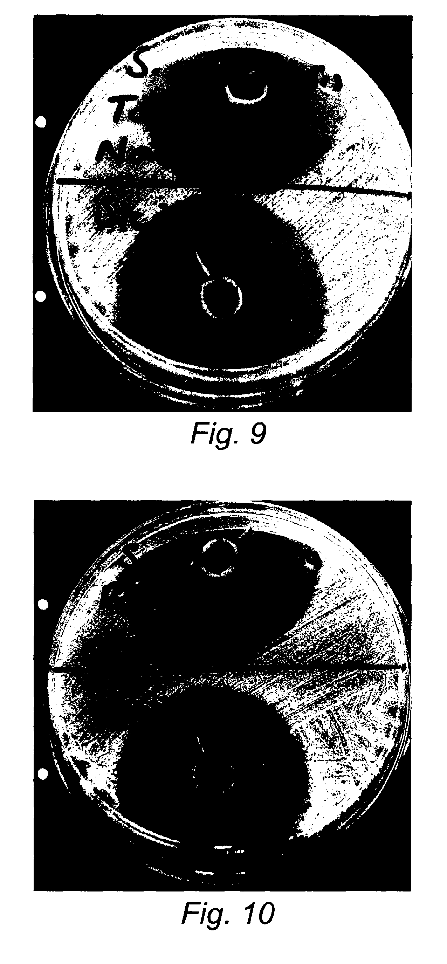

Figures 9 and 10 show a culture of *Staphylococcus epidermidis* on agar plates. A zone of inhibition can be seen around each coated pieces of graft.
Figure 9 shows a graft coated with rifampicin/tobramycin. The zone size recorded was 50mm. Figure 10 shows a graft coated with rifampicin/minocycline. The zone size recorded was 34mm.

### Example 5

A synerginistic response was observed with the rifampicin/tobramycin combination for the MIC/MBC of *Escherichia coli.*

The term "MIC" refers to the lowest concentration of antibiotic that inhibits *in vitro* bacterial growth. The term "MBC" refers to the lowest concentration of antibiotic that totally kills 99.9% of the inoculum.

The principle of the test is to add dilutions of an antibiotic to Mueller-Hinton Broth and a standard concentration of the inoculum. Tubes are incubated overnight and read visually for turbidity. The lowest concentration showing no visible growth is the MIC. To establish the MBC broth cultures showing no visible growth are sub-cultured onto appropriate agar and incubated to determine if the organism has been killed.

The MIC/MBC results for *Escherichia coli* are given in Table 1 below.

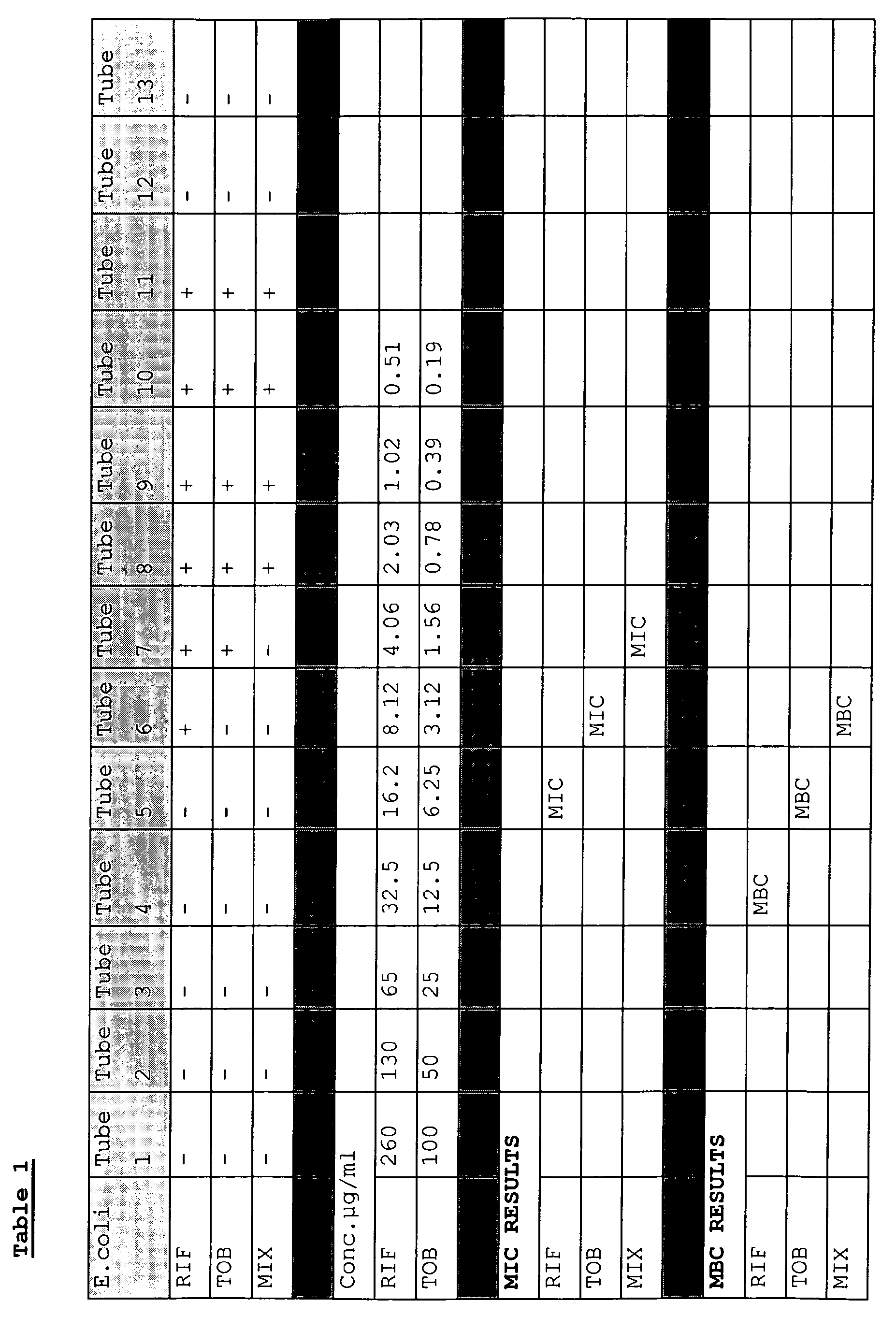

From the results the following can be concluded:

| | MIC | MBC |
|---|---|---|
| Rifampicin | 16.2µg/ml | 32.5µg/ml |
| Tobramycin | 3.12µg/ml | 6.25µg/ml |
| Mixed Antibiotic | 4.06/1.56µg/ml | 8.12/3.12µg/ml |

Rifampicin, alone, had a MIC of 16.2 µg/ml and a MBC of 32.5 µg/ml.

Tobramycin, alone, had a MIC of 3.12 µg/ml and a MBC of 6.25 µg/ml.

In combination, the MIC is 4.06 µg/ml for rifampicin and 1.56 µg/ml for tobramycin. In combination, the MBC is 8.12 µg/ml for rifampicin and 3.12 µg/ml for tobramycin.

The MIC (minimum inhibitory concentration) and MBC (minimum bactericidal concentration) for rifampicin, tobramycin and the drugs in combination were established against a variety of the test organisms.

These results demonstrate that in combination these two particular antibiotics show synergy. This is because the concentration of total antibiotic required to kill the organism is less when in combination than when used individually.

### Example 6

The durability of the antibacterial effect was demonstrated by placing vascular grafts (made according to Example 1) in a flow loop to simulate the washout effect of the blood flow after implant. After 2 days the grafts were still active and showed significant zones of inhibition.

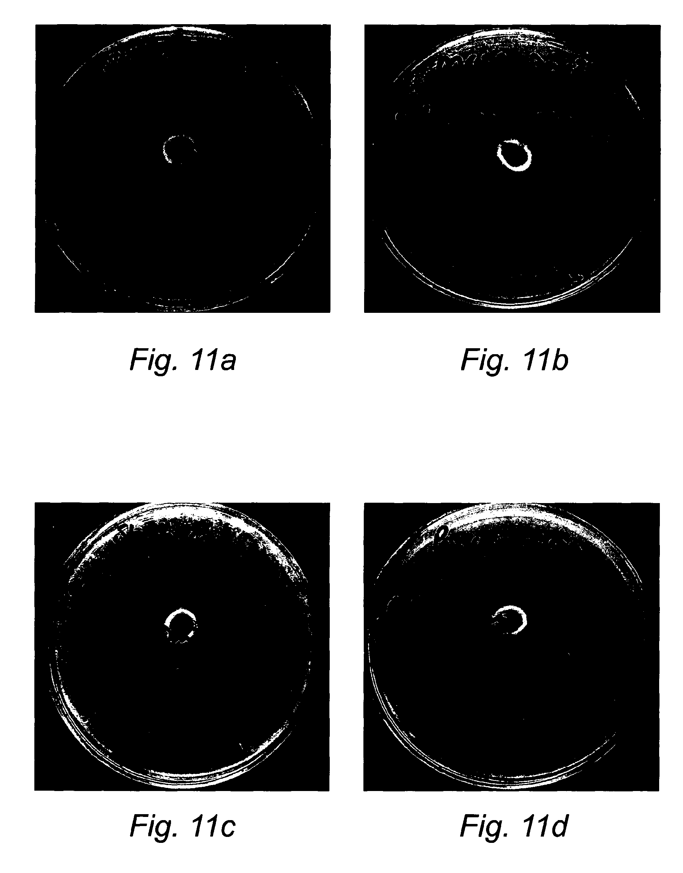

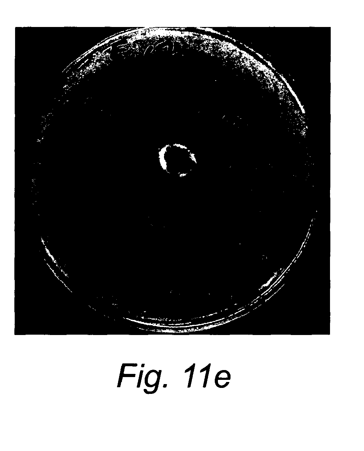

A full length antibiotic coated vascular graft was placed in a flow through loop attached to 5 litres of blood substitute fluid. The blood substitute was recirculated through the graft for a number of days. Figure 11 shows the results when sections of this vascular graft were removed after 2 days and placed on an agar plate containing either S. *aureus,* MRSA, *S. epidermis, Ps. aeruginosa* or *E. coli* bacteria. The clear zone around the graft is the area where the bacteria are being killed by the graft.

These results show that after two days elution, the antibiotic coated grafts are still effectively preventing the growth of the bacteria.

An implantable device which has a combination of rifampicin and tobramycin incorporated into crosslinked gelatin is effective at killing a wide range of bacteria. The effect persists for at least two days. The combination is superior to rifampicin plus minocycline and shows synergy.

### Example 7

The safety and effectiveness of vascular prostheses of the invention in preventing bacterial infection was evaluated in a dog model. The study was conducted in three parts i. bacterial challenge; ii. healing, safety and toxicity of grafts with nominal concentrations of antibiotics and a double dose of antibiotics; and iii. measurement of the antibiotics in the serum of the dog and on the graft over the period of implantation.

### Method

### i. Bacterial Challenge

Twelve 6 mm Gelsoft Plus grafts were implanted into dogs end to end in the infrarenal aorta. Six of these grafts had rifampicin/tobramycin present in the gelatine and were prepared as described in Example 1, and six of the grafts were gelatin coated controls only. Immediately after implantation, 6 x 10⁴ *Staphylococcus aureus* was applied topically directly onto the graft. One week after implantation, the grafts were sterilely harvested. Quantitative cultures were obtained from the harvested grafts and results were expressed as colony forming units per half graft. The results of the bacterial challenge tests are summarised in Table 2.

### ii. Healing Safety and Toxicity

The healing safety and toxicity study was carried out in three parts.

### Part 1 - Histological Examination of grafts

Thirteen 6 mm Gelsoft Plus grafts were implanted into dogs end to end in infrarenal aorta. Six of the grafts were non-antibiotic controls and six were coated with rifampicin and tobramycin and were prepared as described in Example 1. Two dogs with control grafts and two dogs with antibiotic grafts were sacrificed at week 1 post implant and two control dogs and three test dogs were sacrificed at week 3 post implant. The remaining 4 dogs were sacrificed at weeks 12 or 13 post implant. All of the dogs were alive at the time of harvesting without any symptoms of toxicity. The grafts were sterilely harvested and examined histologically, in addition the two control grafts which were harvested at week 3 were sectioned longitudinally. One longitudinal half was used for histology, the other half was used as a control for antibiotic quantification of the harvested antibiotic grafts. The histology examined the healing of the graft and any local toxicity caused by the graft during implantation.

### Part 2 - Histological Examination of Double dose, Worst Case Antibiotic Grafts

Four grafts were prepared using the procedure of Example 1 with twice the nominal dose of antibiotics. These grafts were designed to relate to the worst case scenario. These grafts were implanted into dogs end to end in the infrarenal aorta. Two of these dogs were sacrificed at week 3 post implant, and the remaining two were sacrificed at week 13 post implant. The grafts were harvested sterilely and examined for histology. The histology examined the healing of the graft and any local toxicity caused by the graft during implantation.

### Part 3 - Liver / Kidney Function Tests

Four 6 mm Gelsoft Plus grafts were implanted into dogs end to end in the infrarenal aorta. Two of these grafts had rifampicin/tobramycin present in the gelatin (prepared in accordance with Example 1) and two were gelatin coated controls. At specified time periods during the study (Days 1, 3, 7, 14 and 21 post-implant), enough blood from each dog was removed to provide two, 1 ml samples of serum. The following liver and kidney function tests were carried out on the samples of serum.

### Liver Function

Alanine Aminotransferase (ALT)
Aspartate Aminotransferase (AST)
Alkaline Phosphatase (AP)
Gamma Glutamyl Transferase (GGT)
Bilirubin (BIL)

### Kidney Function

Blood Urea Nitrogen (BUN)
Creatinine (CREAT)

These were carried out using Menarini® TARGA equipment using photometric measurements in ORBIO laboratory (69330 JONAGE). The results are summarised in Table 3 and Table 4.

### iii. Elution Rate

The elution rate was broken down into two parts.

### Part 1 - Determination of rifampicin/tobramycin eluted from the graft with time.

Serum samples were collected from the dogs at the same time as the samples for liver and kidney function tests. The analytical methods for rifampicin and tobramycin are shown below.

### Part 2 - Determination of the rifampicin/tobramycin content remaining on the graft with time.

Six 6 mm Gelsoft Plus grafts were implanted into dogs end to end in the infrarenal aorta. These grafts had rifampicin/tobramycin present in the gelatin of the grafts. Two grafts were harvested after week 1, two grafts after week 2 and the final two grafts were harvested after week 3. Immediately after harvesting, the grafts were prepared for antibiotic analysis. The results are summarised in Table 5 and Table 6 below.

### Analytical Methods for Rifampicin and Tobramycin

### Rifampicin

The analytical method for graft samples and serum samples was identical. The method used a HPLC system with a Hypersil 5 ODS column with UV detection at a wavelength of 254 nm. The mobile phase used was a 70:30 0.2M phosphate buffer pH 6.0:acetonitrile.
The limit of detection for this method is 0.1 mg/Kg graft or 0.1 mg/litre serum.

### Serum samples

These samples were mixed 50:50 with methanol and left to stand for 1 minute. After this time the solution was centrifuged for 5 minutes at maximum speed in a Microcentaur centrifuge. The supernatant was then used for analysis.

### Graft Samples

The graft samples required the rifampicin to be extracted from the graft material. The extraction procedure was as follows.

A volume of methanol equivalent to 3 times the dry weight of the graft was added. Rifampicin was extracted at 4°C for 5 hours, before being centrifuged at 10,000 g. The supernatant was then injected directly into the HPLC.

This extraction process was repeated on the same graft sample to ensure that all the rifampicin had been extracted.

### Tobramycin

The analytical method for serum samples and graft samples was identical. The technique used to measure the tobramycin was Polarisation Fluorolmmuno Assay (PFIA) using an Abbott FLX instrument using the reverse dilution protocol with a sample volume of 5µl. The limit of detection for this method is 0.4mg/Kg graft or 0.4mg/litre serum.

### Serum samples

The serum samples were analysed directly using the Abbot FLX instrument.

### Graft samples

The graft samples required the tobramycin to be extracted from the graft material. The extraction procedure was as follows.

A volume of 2 % trypsin equivalent to 3 times the wet weight of the graft samples was added. The tobramycin was then extracted at 4°C for 5 hours, before being centrifuged at 10,000 g. The supernatant was then injected directly into the PFIA.

The extraction was repeated on the same graft sample to ensure that all the tobramycin had been extracted.

### Results

**Table 2 - Bacterial Challenge Results**

| Dog ID | Inoculum Concentration (CFU/ml) | Number of bacteria on graft after explant (UFC/ Half graft) | Number of Bacteria in organs | | | | |
|---|---|---|---|---|---|---|---|
| | | | Lung | Spleen | Liver | Kidney | PP tissue |
| Challenge Control Dog 1 | 1×10⁴ | 4×10³ | + | + | + | - | + |
| Challenge Control Dog 2 | 8×10³ | 3×10² | + | + | + | + | + |
| Challenge Control Dog 3 | 6×10³ | - | - | - | - | - | - |
| Challenge Control Dog 4 | 6×10³ | 6×10⁶ | - | - | - | - | + |
| Challenge Control Dog 5 | 2×10⁴ | 1×10¹⁰ | + | + | + | + | + |
| Challenge Control Dog 6 | 2×10⁴ | 4×10⁵ | + | + | + | - | + |
| Challenge Test Dog 1 | 1×10⁴ | - | - | - | - | - | - |
| Challenge Test Dog 2 | 2×10⁴ | - | - | - | - | - | - |
| Challenge Test Dog 3 | 2×10⁴ | - | - | - | - | - | - |
| Challenge Test Dog 4 | 2×10⁴ | - | - | - | - | - | - |
| Challenge Test Dog 5 | 2×10⁴ | - | - | - | - | - | - |
| Challenge Test Dog 6 | 2×10⁴ | - | - | - | - | - | - |

### Serum Chemistry

**Table 3 - Liver Function Test Results**

| **Dog** | **ALT (IU/litre)** | **AST (IU/litre)** | **AP (IU/litre)** | **GGT (IU/litre)** | **BIL (mg/l)** |
|---|---|---|---|---|---|
| Elution Control Dog 1 Day 1 | 44 | 127 | 91.3 | 4.0 | 5.8 |
| Elution Control Dog 2 Day 1 | 34.5 | 133 | 18 | 2.0 | 5.8 |
| Elution Control Dog 1 Day 3 | 43.2 | 47.7 | 77.8 | 2.3 | 5.8 |
| Elution Control Dog 2 Day 3 | 30.2 | 41.8 | 98.7 | 3.7 | 5.9 |
| Elution Control Dog 1 Day 7 | 32.6 | 18.4 | 77.1 | 3.6 | 5.8 |
| Elution Control Dog 2 Day 7 | 25.7 | 19.3 | 80 | 2.8 | 6.1 |
| Elution Control Dog 1 Day 14 | 19.3 | 15 | 80.1 | 3.4 | 5.6 |
| Elution Control Dog 2 Day 14 | 12.8 | 21.1 | 125 | 2.6 | 5.9 |
| Elution Control Dog 1 Day 21 | 20.3 | 14.6 | 61.8 | 3.1 | 5.9 |
| Elution Control Dog 2 Day 21 | 13.5 | 25 | 120 | 4.0 | 6.0 |
| Elution Test Dog 1 Day 1 | 15.3 | 32.3 | 43.8 | 2.3 | 5.6 |
| Elution Test Dog 2 Day 1 | 41.1 | 103 | 36.6 | 5.6 | 7.3 |
| Elution Test Dog 1 Day 3 | 12.9 | 21.6 | 50.9 | 3.0 | 5.5 |
| Elution Test Dog 2 Day 3 | 37.5 | 27 | 114 | 6.6 | 5.8 |
| Elution Test Dog 1 Day 7 | 9.7 | 9.8 | 40.2 | 3.3 | 5.5 |
| Elution Test Dog 2 Day 7 | 29.7 | 17.8 | 96.4 | 5.5 | 5.9 |
| Elution Test Dog 1 Day 14 | 5.8 | 15 | 46.7 | 3.0 | 5.7 |
| Elution Test Dog 2 Day 14 | 15.2 | 15.8 | 181 | 4.0 | 5.9 |
| Elution Test Dog 1 Day 21 | 7.9 | 16.6 | 54.4 | 3.0 | 6 |
| Elution Test Dog 2 Day 21 | 25.1 | 24.4 | 219 | 5.1 | 6.2 |

**Table 4 - Kidney Function Test Results**

| **Dog** | **BUN (g/litre)** | **CREAT (mg/litre)** |
|---|---|---|
| Elution Control Dog 1 Day 1 | 0.3 | 8.5 |
| Elution Control Dog 2 Day 1 | 0.1 | 8 |
| Elution Control Dog 1 Day 3 | 0.4 | 9.7 |
| Elution Control Dog 2 Day 3 | 0.3 | 8.2 |
| Elution Control Dog 1 Day 7 | 0.4 | 9.8 |
| Elution Control Dog 2 Day 7 | 0.3 | 9.1 |
| Elution Control Dog 1 Day 14 | 0.3 | 9.1 |
| Elution Control Dog 2 Day 14 | 0.3 | 7.4 |
| Elution Control Dog 1 Day 21 | 0.3 | 7.7 |
| Elution Control Dog 2 Day 21 | 0.2 | 5.9 |
| Elution Test Dog 1 Day 1 | 0.1 | 7.3 |
| Elution Test Dog 2 Day 1 | 0.2 | 13.8 |
| Elution Test Dog 1 Day 3 | 0.3 | 9.5 |
| Elution Test Dog 2 Day 3 | 0.5 | 11.5 |
| Elution Test Dog 1 Day 7 | 0.3 | 9.3 |
| Elution Test Dog 2 Day 7 | 0.4 | 10.8 |
| Elution Test Dog 1 Day 14 | 0.3 | 9.3 |
| Elution Test Dog 2 Day 14 | 0.4 | 10.5 |
| Elution Test Dog 1 Day 21 | 0.3 | 8.5 |
| Elution Test Dog 2 Day 21 | 0.3 | 8.4 |

### Liver Function

### Alanine amino-transferase (ALAT)

The values obtained were within the physiological range, and similar between test and control dogs.

### Aspartate amino-transferase (AST)

Aspartate amino-transferase rates were out of the physiological range for the control dogs 7 and 8 on day 1 and for the test dog 8 on day 1. These increased rates were sporadic events.

### Alkaline phosphatase (AP)

Alkaline phosphoatase rates were out of physiological range for the control dog 8 on day 1 and for the test dog 8 on day 1 and day 14. These increased rates were sporadic events.

### Gamma glutamyl amino-transferase (γ-GT)

The values obtained were within the physiological range, and similar between test and control dogs.

### Bilirubin (BIL)

Higher bilirubin rates, were out of physiological range for the test and control dogs, regardless of the time period. The rates were similar between both test and control animals.

### Kidney Function

### Blood urea nitrogen (BUN)

Blood urea nitrogen rates were out of physiological range for the control dogs 7 and test dog 8 on day 1 and for the test dog 8 on day 1. These decreased rates were sporadic events.

### Serum creatinine (CREAT)

Serum creatinine rate was out of physiological range for the test dog 8 on day 1 and for the test dog 8 on day 1 and 14. These decreased rates were sporadic events.

Under the conditions of this study and in the absence of any other toxicology data, no evidence of hepatocellular, biliary or renal dysfunction was observed.

### Histology

In prostheses harvested one week post-implant, no fibrous organization was observed. In some of these grafts, a granulation tissue surrounded the prosthesis. The prosthetic wall was preserved and presented acute inflammation with polymorphonuclear cells (PMN) in variable amount. Mural thrombus was constant, but had variable thickness. No differences were observed between control grafts and antibiotic loaded grafts after one week of implantion.

In prostheses harvested three weeks post-implant, no fibrous organization was observed. The prostheses were surrounded by a granulation tissue. The amount of PMN dramatically decreased, except in one prosthesis, with abscess formation. Some giant cell appeared, as often observed with polyester grafts.

No differences were observed between the grafts loaded with nominal concentrations of antibiotics and the grafts loaded with twice the optimal concentration.
In prostheses harvested 12-13 weeks post-implant, a fibrous organization developed resulting in the formation of an adherent peripheral capsule with giant cells. The structure of the prostheses was preserved with absence or minimal fibrous ingrowth from the periphery. Some giant cells were observed within the prosthesis. A neointima developed in most of the prosthesis, but was inconstant, as well as thrombus, more frequently observed in areas without neointima. No differences were observed between the grafts loaded with nominal concentrations of antibiotics and the grafts loaded with twice the optimal concentration.

The findings are consistent with the normal healing of a gelatin sealed vascular graft, at the different stages of explantation. No abnormality was found in the different samples analysed.

### Elution Rate

**Table 5 - Drug Elution Results (Serum Samples)**

| **Dog ID** | **Implant Duration** | **Tobramycin Content (mg/litre serum)** | **Rifampicin Content (mg/litre serum)** |
|---|---|---|---|
| Elution Control Dog 1 | Day 1 | <0.4 | <0.1 |
| | Day 3 | <0.4 | <0.1 |
| | Day 7 | <0.4 | <0.1 |
| | Day 14 | <0.4 | <0.1 |
| | Day 21 | <0.4 | <0.1 |
| Elution Control Dog 2 | Day 1 | <0.4 | <0.1 |
| | Day 3 | <0.4 | <0.1 |
| | Day 7 | <0.4 | <0.1 |
| | Day 14 | <0.4 | <0.1 |
| | Day 21 | <0.4 | <0.1 |
| Elution Test Dog 1 | Day 1 | <0.4 | 0.23 |
| | Day 3 | <0.4 | <0.1 |
| | Day 7 | <0.4 | <0.1 |
| | Day 14 | <0.4 | <0.1 |
| | Day 21 | <0.4 | <0.1 |
| Elution Test Dog 2 | Day 1 | <0.4 | 0.23 |
| | Day 3 | <0.4 | <0.1 |
| | Day 7 | <0.4 | <0.1 |
| | Day 14 | <0.4 | <0.1 |
| | Day 21 | <0.4 | <0.1 |

**Table 6 - Drug Elution Results (Graft Samples)**

| **Dog Number** | **Implant Duration** | **Tobramycin Content (mg/Kg graft)** | | | **Rifampicin Content (mg/Kg graft)** | | |
|---|---|---|---|---|---|---|---|
| | | **1**^{**st**} **Extraction** | **2**^{**nd**} **Extraction** | **Total** | **1**^{**st**} **Extraction** | **2**^{**nd**} **Extraction** | **Total** |
| Elution Control Dog 1 | 21 Days | <0.4 | <0.4 | <0.4 | <0.1 | <0.1 | <0.1 |
| Elution Control Dog 2 | 21 Days | <0.4 | <0.4 | <0.4 | <0.1 | <0.1 | <0.1 |
| Elution Test Dog 6 | 7 Days | 4.8 | <0.4 | 4.8 | 0.89 | <0.1 | 0.89 |
| Elution Test Dog 3 | 7 Days | 3.96 | <0.4 | 3.96 | 0.94 | <0.1 | 0.94 |
| Elution Test Dog 4 | 14 Days | 0.51 | <0.4 | 0.51 | 0.73 | <0.1 | 0.73 |
| Elution Test Dog 5 | 14 Days | <0.4 | <0.4 | <0.4 | 1.24 | <0.1 | 1.24 |
| Elution Test Dog 1 | 21 Days | <0.4 | <0.4 | <0.4 | 0.34 | <0.1 | 0.34 |
| Elution Test Dog 2 | 21 Days | <0.4 | <0.4 | <0.4 | 0.52 | <0.1 | 0.52 |

### Discussion

### Bacterial Challenge (Table 2)

Five of the six control dogs tested positive for *S.auerus* after explantation. Five of the six control dogs showed evidence of infection in the peri-prosthestic tissue and 4 control dogs had *S.aureus* infection in the explanted lung, spleen and liver and two dogs had infection in the kidney.

The six test dogs all showed no evidence of infection after explant, in addition none of the organs or periprosthetic tissue of the tests dogs showed any infection due to the bacteria which were inoculated onto the grafts.

### Healing Safety & Toxicity

### Histology

The examination of all the retrieved graft samples showed a similar healing pattern for both material, i.e. gelatin sealed control grafts and antibiotic loaded grafts. The gelatin disappeared within similar periods of time. With no gelatin present from 21 days in both groups of dogs. At 3 months healing of antibiotic loaded grafts was similar to that observed in control grafts, with a regular fibrous organization of the prosthetic wall. Inflammatory reaction was not increased in test grafts for any duration of implantation. No difference was observed between grafts that were loaded with nominal concentrations of antibiotics and those which had twice the optimal concentrations of antibiotics in the gelatin. Overall, the behavior of antibiotic loaded grafts was strictly similar to that of standard gelatin sealed grafts.

### Liver & Kidney Function Tests (Tables 3 & 4)

### Alanine Aminotransferase (ALT)

The physiological range of ALT as reported by Biomatech is less than 50 IU/litre.

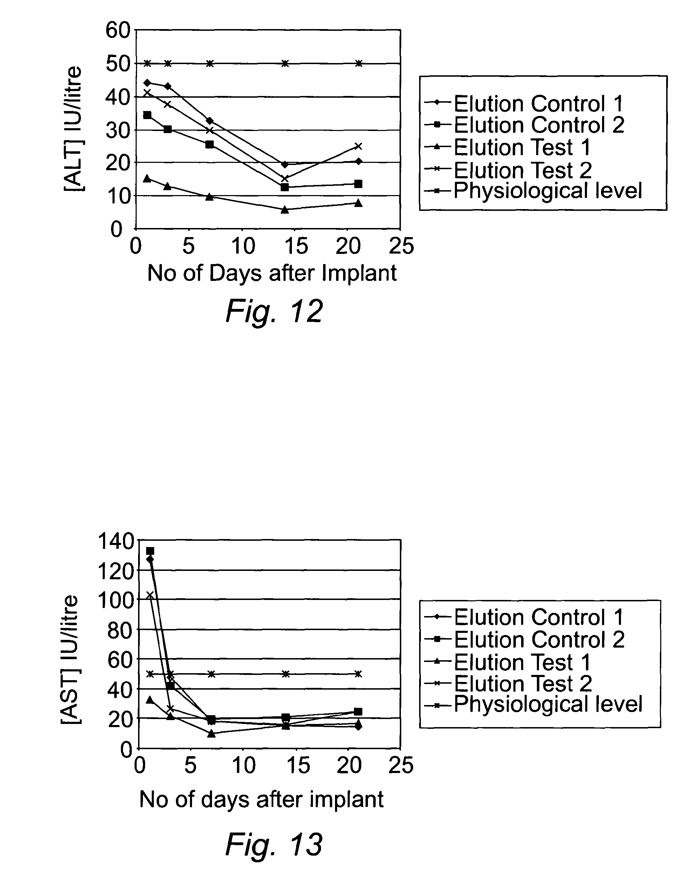

The results in Table 3 and Fig 12 show that all the dogs were within the physiological range and similar between test and control dogs.

ALT is found within the cytoplasm of hepatocytes, it is released into the bloodstream during changes to the permeability of the cell membrane or necrosis.

### Aspartate Aminotransferase (AST)

The physiological range of AST as reported by Biomatech is less than 50 IU/litre.

The results in Table 3 and Fig 13 show the two control dogs and elution test dog 2 had elevated levels which were out of the physiological range.

AST is found in the cytoplasm and mitochondria of hepatocytes, it is released into the bloodstream due to hepatucellular damage and during changes in cell membrane permeability or necrosis.

### Alkaline Phosphatase (AP)

The physiological range of AP as reported by Biomatech is between 30 and 230 IU/litre.

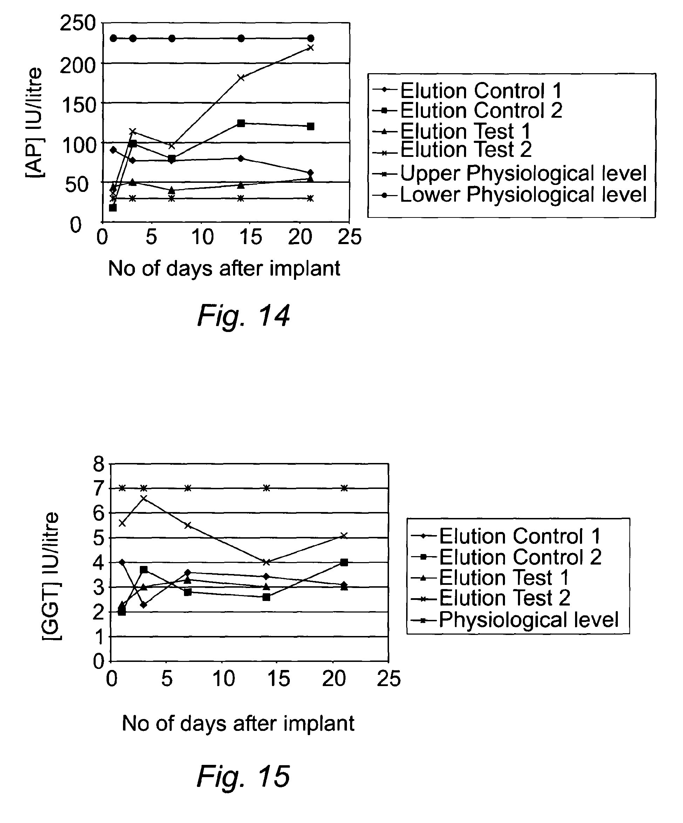

The results in Table 3 and Fig 14 show that the level of AP in elution control dog 2 day 1 was slightly decreased.

Increased serum levels of AP are due to increased synthesis of the enzyme. AP levels will remain elevated during hepatic repair and is not a good prognostic indicator as it is released by healthy hepatocytes.

### Gamma Glutamyl Transferase (GGT)

The physiological range of GGT as reported by Biomatech is less than 7 IU/litre.

The results in Table 3 and Fig 15 show that all the control and test dogs had GGT levels which were within this limit and that the results between the control and test dogs were similar. The highest concentration of GGT is found in the renal tubule cells. However, as it is excreted in the urine, levels do not rise as a response to renal damage.

### Bilirubin (BIL)

The physiological range of BIL as reported by Biomatech is between 0.4 and 3.4 mg/litre.

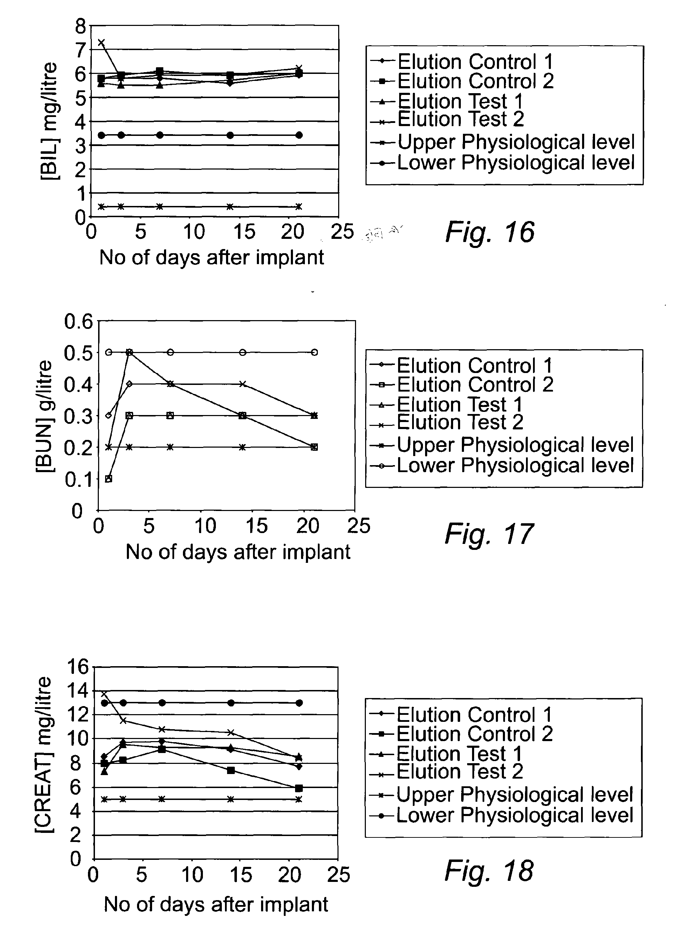

The results in Table 3 and Fig 16 show that the bilirubin levels in the control and test dogs are all higher than the physiological range. The results between the control and test dogs are similar. Bilirubin is formed from the metabolism of haem groups in the liver, spleen and bone marrow and is taken up and conjugated by hepatocytes before excretion in the bile.

### Blood Urea Nitrogen (BUN)

The physiological range of BUN as reported by Biomatech is between 0.2 and 0.5 g/litre.

The results in Table 4 and Fig 17 show that the BUN levels in elution control dog 2 and elution test dog 1 at day 1 were decreased.

Urea is principally a product of amino acid deamination in the liver. It is primarily excreted in the kidneys and is the most common test of renal function.

### Creatinine (CREAT)

The physiological range of creatinine as reported by Biomatech is 5 to 13mg/litre.

The results in Table 4 and Fig 18 show that creatinine levels were over this range for elution test dog 2 on day 1.
Creatinine is produced at a steady rate due to muscle catabolism and is not reabsorbed by the kidney tubules after filtration. Its measurement gives an indirect measurement of glomerular filtration rate. Unlike BUN, the levels of creatinine are not markedly affected by diet, or any aspect of liver function and therefore the test is more specific for renal dysfunction.

### Drug Elution Results (Table 5 & 6)

### Rifampicin/ Tobramycin Content of serum (Table 5)

The drug elution results show that there is no detectable amount of tobramycin present in the serum of the test dogs at any time point in the elution. The rifampicin could only be detected at day one of the elution, at a level of 0.23 mg/litre of serum. If this was equated to an average human with a blood volume of 5 litres, the quantity of rifampicin would be 1.15 mg. This is much less than the maximum allowable quantity of rifampicin.

As expected, none of the serum from the elution rate control grafts had any detectable amounts of antibiotics present at any time point.

### Rifampicin/Tobramycin content of graft with time (Table 6)

There were no detectable amounts of rifampicin or tobramycin present in any of the grafts from the control group.

The results show that the rifampicin is detectable in small quantities on the grafts at up to and including day 21. Tobramycin was detectable on the two test grafts after 7 days implant time and in one of the test dogs after 14 days implant.

### Conclusion

### Bacterial Challenge

The results show that the method of inoculation and the size of the inoculum were sufficient to cause infection in five of the six control dogs.

When the antibiotic coated test grafts were inoculated with the same bacteria at the same concentration using the same method of inoculation, no infection was found in any of the test dogs.

### Healing, Safety and Toxicity

### Histology

The examination of all the retrieved graft samples showed a similar healing pattern for both materials, i.e. gelatin sealed control grafts and antibiotic loaded grafts. The gelatin disappeared within similar periods of time, with no gelatin present from 21 days in both groups of dogs. At 3 months healing of antibiotic loaded grafts was similar to that observed in control grafts, with a regular fibrous organization of the prosthetic wall. Inflammatory reaction was not increased in test grafts for any duration of implantation. No difference was observed between grafts that were loaded with nominal concentrations of antibiotics and those which had twice the optimal concentrations of antibiotics in the gelatin. Overall, the behavior of antibiotic loaded grafts was strictly similar to that of standard gelatin sealed grafts.

### Serum Chemistry

### Liver Function

The results show that the test dogs had no significant deterioration in liver function when compared to the control dogs. In fact, for some of the tests i.e. ALT and ASP, the results for the test dogs were better than those of the control dogs. All dogs, control and test had elevated bilirubin results, the result for each dog was very similar, and the higher than normal results may have been as a result of the surgical procedure rather than the graft type.

There is no evidence of any decrease in liver function in either the test or control dogs.

### Kidney Function

The results show that the results for Urea were very similar in both the control and test dogs. The Creatinine test which is a better test for kidney function shows that the only difference between the control and test dogs was a slightly higher than normal result at day 1 for one of the test dogs. This level soon stabilized and returned to expected levels for the duration of the implant.

There is no evidence of any decrease in kidney function for either the test or control dogs.

No evidence of hepatocellular of biliary dysfunction was observed. No evidence of renal dysfunction was observed.

### Medical examination results

### Healing safety and Toxicity

Medical examination results were included in the Intensive Care forms for each of the dogs.

The results of the physical and physiological checks carried out on the control and test dogs showed that the antibiotic coating had no adverse effects on any of the parameters measured (state of consciousness, temperature, pulse, respiratory frequency, colour of mucous membrane, TRC, skin elasticity, appetite, urine output, bowel movement and weight) during the course of the implantation.

### Elution Rate

### Drug Elution (Serum Samples)

There was no detectable tobramycin present in the serum of any of the test dogs at any of the time points of the implant. Rifampicin was only detectable in the serum of the test dogs at day 1 of the implantation period.

### Drug Elution (Graft)

The tobramycin is not present at detectable levels on the graft after 14 days implantation. The rifampicin is still present 21 days after implantation.

## Claims

1. A medical device which is impregnated with an antibiotic composition or which has at least one surface at least partially coated with an antibiotic composition, said antibiotic composition comprising rifampicin and tobramycin.

2. The medical device of Claim 1 which has at least one surface at least partially coated with said antibiotic composition.

3. The medical device of Claim 1 which is impregnated with said antibiotic composition.

4. The medical device as claimed in any one of Claims 1 to 3 which is for implantation within a patient.

5. The medical device is claimed in Claim 4 which is a vascular graft, a vascular patch or a heart valve.

6. The medical device as claimed in any one of Claims 1 to 5 wherein said device is sealed with a sealant.

7. The medical device as claimed in Claim 6 wherein said sealant is gelatin, collagen, cross-linked dextran, polysaccharide, albumin, hyaluronic acid, chitosan or synthetic hydrogel.

8. The medical device as claimed in Claim 7 wherein said sealant is a gelatin sealant.

9. The medical device as claimed in any one of Claims 6 to 8 wherein said sealant is plasticised with a non-volatile non-toxic plasticiser.

10. The medical device as claimed in Claim 9 wherein the plasticiser is glycerol or sorbitol.

11. The medical device as claimed in any one of Claims 1 to 10 wherein 0.05 to 0.15mg of rifampicin is present per cm² of graft.

12. The medical device as claimed in Claim 11 wherein 0.08 to 0.10mg of rifampicin is present per cm² of graft.

13. The medical device as claimed in any one of Claims 1 to 12 wherein 0.01 to 0.05mg of tobramycin is present per cm² of graft.

14. The medical device as claimed in Claim 13 wherein 0.02 to 0.04mg of tobramycin is present per cm² of graft.

15. The medical device as claimed in any one of Claims 1 to 10 which contains amounts of rifampicin and tobramycin effective against infections caused by *Staphylococcus* bacteria.

16. The medical device as claimed in Claim 15 wherein said bacteria are *Staphylococcus aureus* or *Staphylococcus epidermidis.*

17. A method of preparing an infection resistant medical device, said method comprising:
i) treating at least part of said medical device with a sealant to produce a treated medical device;
ii) exposing said treated medical device to a solution of rifampicin and tobramycin or to two solutions, one of which contains rifampicin and the other of which contains tobramycin.

18. The method as claimed in Claim 17 wherein the sealant of step i) is gelatin, collagen, cross-linked dextran, polysaccharide, albumin, hyaluronic acid, chitosan or synthetic hydrogel.

19. The method is claimed in Claim 18 wherein the sealant is glycerol.

20. The method as claimed in any one of Claims 17 to 19 wherein a non-volatile non-toxic plasticiser is present in combination with the sealant of step i).

21. The method as claimed in Claim 20 wherein the plasticiser is glycerol or sorbitol.

22. The method as claimed in either one of Claims 20 an 21 wherein a plasticiser is present in the solution of step ii).

23. The method as claimed in Claim 22 wherein the same plasticiser is used in both steps i) and ii).

24. The method as claimed in any one of Claims 17 to 23 wherein the concentration of rifampicin in the solution of step ii) is from 10 g/litre to 15 g/litre.

25. The method as claimed in Claim 24 wherein the concentration of rifampicin is from 11g/litre to 14g/litre.

26. The method as claimed in any one of Claims 17 to 25 wherein the concentration of tobramycin in the solution of step ii) is from 0.5 g/litre to 7 g/litre.

27. The method as claimed in Claim 26 wherein the concentration of tobramycin is from 1g/litre to 6.5g/litre.

28. The method as claimed in any one of Claims 17 to 27 further including the step of sterilising the device.

29. The method as claimed in Claim 28 wherein the device is sterilised using low temperature ethylene oxide gas.

30. A method of inhibiting bacterial growth on a medical device, said method comprising:
a) applying a composition comprising a combination of rifampicin and tobramycin to at least part of said device, said composition being applied at a concentration sufficient to inhibit bacterial growth.

31. The method as claimed in Claim 30 wherein said bacterial growth is due to *Staphylococcus* bacteria.

32. The method as claimed in Claim 31 wherein said bacteria is *Staphylococcus aureus* or *Staphylococcus epidermidis.*

33. A composition comprising rifampicin and tobramycin for use in treating at least part of a medical device in order to inhibit bacterial growth thereon.

34. Use of a composition comprising rifampicin and tobramycin in the manufacture of a medical device which inhibits bacterial growth thereon.
